(19) **Europäisches Patentamt**

**European Patent Office**

**Office européen des brevets**

(11) Veröffentlichungsnummer : **0 314 696 B1**

(12) **EUROPÄISCHE PATENTSCHRIFT**

(45) Veröffentlichungstag der Patentschrift :
**04.03.92 Patentblatt 92/10**

(21) Anmeldenummer : **87904673.8**

(22) Anmeldetag : **13.07.87**

(86) Internationale Anmeldenummer :
**PCT/AT87/00039**

(87) Internationale Veröffentlichungsnummer :
**WO 88/00478 28.01.88 Gazette 88/03**

(51) Int. Cl.⁵ : **A61M 5/34**

(30) Priorität : **11.07.86 AT 1883/86**

(43) Veröffentlichungstag der Anmeldung :
**10.05.89 Patentblatt 89/19**

(45) Bekanntmachung des Hinweises auf die
Patenterteilung :
**04.03.92 Patentblatt 92/10**

(84) Benannte Vertragsstaaten :
**BE CH DE FR GB IT LI LU NL SE**

(56) Entgegenhaltungen :
**WO-A-86/03126**
**DE-A- 2 434 046**

(73) Patentinhaber : **PICKHARD, Ewald**
**Redtenbachergasse 15**
**A-1160 Wien (AT)**

(72) Erfinder : **PICKHARD, Ewald**
**Redtenbachergasse 15**
**A-1160 Wien (AT)**

(74) Vertreter : **Wolke, Heidemarie, Dr.**
**Stadtplatz 7**
**A-4400 Steyr (AT)**

(54) **INJEKTIONSSPRITZE MIT NADELSCHUTZKAPPE.**

Jouve, 18, rue Saint-Denis, 75001 PARIS

EP 0 314 696 B1

EP 0 314 696 B1

## Beschreibung

Die Erfindung bezieht sich auf eine Injektionsspritze, wie sie im Oberbegriff des Patentanspruches 1 beschrieben ist.

Eine bekannte Injektionsspritze - gemäß WO-A-86/03126 - besteht aus einem Spritzenkörper und einem in diesem eingesetzten, die Spritzennadel aufnehmenden Nadelträger. Weiters weist die Injektionsspritze eine mit dem Nadelträger kraft- oder formschlüssig verbundene, mit einem Kupplungsteil des Spritzenkörpers zumindest teilweise formschlüssig kuppelbare Haltevorrichtung auf. Der Spritzennadel ist eine diese umhüllende und sich bis in den Bereich des Nadelträgers erstreckende Nadelschutzkappe zugeordnet. Zwischen dem Nadelträger und dem der Nadelspitze zugeordneten Ende der Nadelschutzkappe ist eine Öffnungsvorrichtung angeordnet. Desweiteren ist zwischen dem Spritzenkörper und dem von diesem abgewendeten Ente der Spritzennadel eine Verschlußvorrichtung für einen Spritzkanal und bzw. oder einen Spritzennadelkanal angeordnet. Dieser Verschlußvorrichtung ist ein innerhalb der Nadelschutzvorrichtung angeordnetes Öffnungsorgan zugeordnet.

Eine weitere bekannte Injektionsspritze - gemäß DE-A-24 34 046 - besteht aus einem Spritzenkörper und einem in diesem eingesetzten die Spritzennadel aufnehmenden Nadelträger. Weiters weist die Injektionsspritze eine mit dem Nadelträger kraft- oder formschlüssig verbundene, mit einem Kupplungsteil des Spritzenkörpers zumindest teilweise formschlüssig kuppelbare Haltevorrichtung auf. Der Spritzennadel ist eine diese umhüllende und sich bis in den Bereich des Nadelträgers erstreckende Nadelschutzkappe zugeordnet. Zwischen dem Spritzenkörper und einem die Spritzennadel aufnehmenden Nadelkörper, ist eine durch eine Dichtscheibe gehildete Verschlußvorrichtung vorgesehen. Der Lagerteil ist mit seinem Außengewinde in ein Innengewinde eines Tragkörpers eingeschraubt, auf dessen Außengewinde die Nadelschutzkappe mit einem Innengewinde aufgeschraubt ist. Zwischen der Nadelschutzkappe und dem Lagerkörper sind ineinandergreifende Mitnahmeorgane vorgesehen. Die Gewinde zwischen dem Tragkörper und der Nadelschutzkappe bzw. dem Tragkörper und dem Lagerteil, sind gegengleich ausgeführt. Durch eine Verdrehung der Nadelschutzkappe vom Spritzkörper weg, wird gleichzeitig über die Mitnahmeorgane der Lagerteil in denselben eingeschraubt und die von der Spitze der Spritzennadel abgewendete Seite durch die Verschlußvorrichtung in den Innenraum des Spritzenkörpers durchgestoßen. Dadurch wird es möglich, durch ein Verdrehen der Nadelschutzkappe die Dichtscheibe zu durchstoßen. Nachteilig ist hierbei, daß durch die Verwendung von gegenläufigen Gewinden bei den Teilen mit sehr kleinen Abmessungen, wie dies bei Injektionsspritzen der Fall ist, sehr häufig Verklemmungen auftreten, dip zu einer Zerstörung der Injektionsspritze oder zu einem gewaltsamen Öffnen mit dem Risiko einer Verletzung der Bedienungsperson führt.

Der Erfindung liegt die Aufgabe zugrunde, eine Injektionsspritze der eingangs genannten Art zu schaffen, die einfach steril verpackt werden kann und die mit einem Originalitätsverschluß versehen ist, sodaß eine Öffnung der sterilen Verpackung sofort erkannt werden kann, wobei darüberhinaus verhindert werden soll, daß eine im Soritzenkörper enthaltene Lösung vor dem Öffnen des Originalitätsverschlusses aus dem Spritzenkörper austreten kann.

Die Aufgabe der Erfindung wird durch die Merkmale im Kennzeichenteil des Patentanspruches 1 gelöst. Der überraschende Vorteil dieser Lösung liegt darin, daß durch die kraft- und formschlüssige Bewegungsverbindung zwischen dem Nadelträger und der Haltevorrichtung bzw. dem Basisteil mit einem kurzen Hub, die Verschlußvorrichtung des Spritzenkanals geöffnet und eine Originalitätssicherung erreicht werden kann. Mummehr ist es durch ein einziges Gewinde und eine einfache Verdrehung eines Teiles der Nadelschutzvorrichtung nämlich den Kappenteiles, ohne Bewegung desselben, parallel zur Längsrichtung der Spritzennadel möglich die Verschlußvorrichtung zu öffnen. Trotzdem ist es dabei aber noch möglich, den Nadelträger und die Nadelschutzkappe mit der Haltevorrichtung als jeweils eigenen Bauteil herzustellen, wobei der Nadelträger nach Fertigung der Nadelschutzkappe in diese eingesetzt werden kann. Trotzdem wird aber erreicht, daß beim Öffnen der Nadelschutzvorrichtung, insbesondere über die Schwächungslinie, also dem Abtrennen des Kappenteiles und während des Eindrehens des Lagerteiles in den Stützteil des Nadelträgers, sich der Stützteil nicht mit dem Lagerteil mitdrehen kann und dadurch ein sicheres Öffnen der Verschlußvorrichtung und das Aufbringen einer ausreichenden Scherkraft gewährleistet ist.

Dieser Lösung liegt aber auch eine überraschende Erkenntnis zu Grunde, nämlich unter Beibehaltung der Position des Kappenteiles nur durch eine Verdrehung desselben, aber ohne Längsbewegung parallel zur Spritzennadel einen Hub des Lagerteiles parallel zur Spritzennadel auszulösen, mit dem die Verschlußvorrichtung geöffnet und gleichzeitig der Lagerteil in Längsrichtung der Spritzennadel zu jedem Zeitpunkt exakt gegenüber der Haltevorrichtung positioniert werden kann. Dazu kommt, daß es mit einer derartig ausgebildeten Injektionsspritze möglich ist, diese für Medikamente die in kurzen zeitlichen Abständen dem Patienten zu verabreichen sind, zu verwenden. Mittels des Kappenteils der nach dem ersten Gebrauch aufgeschoben wird, kann nämlich durch Verdrehen, die Spritzennadel aus dem Spritzenkörper entfernt und vor dem nächsten Soritzvorgang wie-

2

der in diesen eingeführt werden. All dies erfordert lediglich eine Bewegung des Kappenteils parallel zur Spritzennadel und keinen komplizierten Fügevorgang von Feingewinden, wie dies beispielsweise bei der Injektionsspritze nach der DE-A-24 34 046 der Fall ist.

Von Vorteil ist aber auch eine Weiterbildung gemäß Anspruch 2. Durch die Bewegungsverbindung der Haltevorrichtung mit dem Stützteil kann eine ausreichende Schwerkraft zum Abtrennen der Nadelschutzkapoe sichergestellt werden.

Es ist aber auch eine Ausbildung nach Anspruch 3 möglich, die sicherstellt, daß die Verbindung zwischen der Spritzennadel und dem Spritzenkörper einwandfrei hergestellt werden kann und es wird damit gleichzeitig eine Begrenzung der Bewegung des Lagerteiles gegenüber dem Spritzenkörper ermöglicht.

Weitere Vorteile der Erfindung werden bei einer Ausbildung der Injektionsspritze gemäß den weiteren Unteransprüchen erzielt.

Zum besseren Verständnis der Erfindung wird diese im folgenden anhand der in den Zeichnungen dargestellten Ausführungsbeispiele näher erläutert.

Es zeigen:

Fig. 1 eine erfindungsgemäße Injektionsspritze mit angesetzter Madelschutzkappe und unversehrtem Originalitätsverschluß, axial geschnitten, gemäß den Linien I-I in Fig.3;

Fig. 2 die Injektionsspritze nach Fig.1 bei geöffnetem Originalitätsverschluß und geöffneter Verschlußvorrichtung zwischen dem Spritzenkörper und der Spritzennadel sowie geschnitten, gemäß den Linien I-I in Fig.3;

Fig. 3 die Injektionsspritze nach Fig.1 und 2 in Stirnansicht, geschnitten, gemäß den Linien III-III in Fig.1.

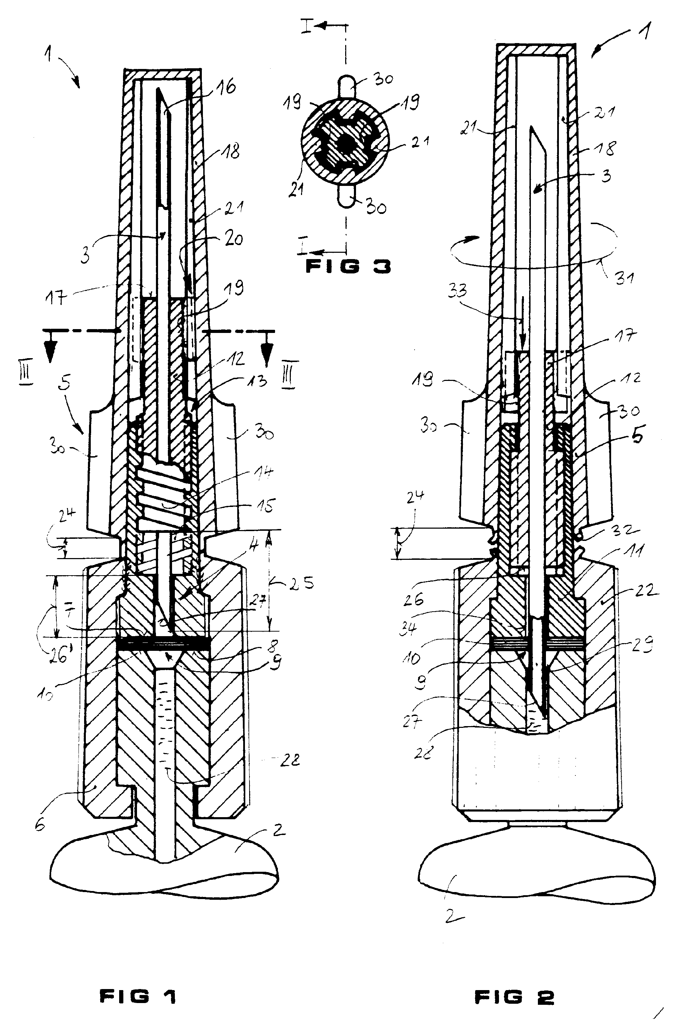

In den Fig.1 bis 3 ist eine Injektionsspritze 1 gezeigt, die einen beispielsweise aus Glas bestehenden Spritzenkörper 2 und eine Spritzennadel 3 aufweist. Die Spritzennadel 3 ist in einem Nadelträger 4 gehaltert, der von einer Nadelschutzkappe 5 umgeben ist und mit einer Haltevorrichtung 6 mit dem Spritzenkörper 2 gekuppelt ist. Zwischen einer Stirnseite 7 des Nadelträgers 4 und einer dieser zugewandten und gegenüberliegenden Stirnseite 8 des Spritzenkörpers 2 ist eine Verschlußvorrichtung 9, beispielsweise eine Dichtscheibe 10 aus Pharmagummi, angeordnet. Der Nadelträger 4 besteht aus einem Stützteil 11 und einem gegenüber diesem verstellbaren Lagerteil 12. Dieser Lagerteil 12 haltert die Spritzennadel 3, in dem die Spritzennadel 3 in den Lagerteil 12 eingespritzt ist. Die Spritzennadel 3 kann aber auch eingepreßt oder eingeklebt sein.

Zur Führung und Halterung des Lagerteiles 12 im Stützteil 11 ist zwischen diesem eine Führungsvorrichtung 13 angeordnet, die im gezeigten Ausführungsbeispiel durch ein am Lagerteil 12 angeordnetes Außengewinde 14 und ein im Stützteil 11 angeordnetes Innengewinde 15 gebildet ist. Der Lagerteil 12 ist weiters mit einem in Richtung eines offenen Endes 16, außerhalb der Führungsvorrichtung 13 angeordneten Fortsatz 17 versehen. Dieser Fortsatz 17 weist an seiner einem Kappenteil 18 der Nadelschutzkappe 5 zugewandten Oberfläche Vertiefungen 19 einer Mitnahmevorrichtung 20 auf, die weiters auf der dem Nadelträger 4 zugewandten Oberfläche angeordnete Mitnahmeleisten 21 umfaßt.

Zwischen dem Kappenteil 18 und einem Basisteil 22 ist ein eine Öffnungsvorrichtung bildender Sollbruchbereich 24 angeordnet. Die Nadelschutzkappe 5 weist in diesem Sollbruchbereich eine dünnere Wandstärke auf als in den übrigen Bereichen. Weiters ist der Stützteil 11 mit dem Basisteil 22 zwischen der Dichtscheibe 10 und dem Sollbruchbereich 24 drehfest verbunden, wie dies schematisch durch eine Schweißnaht angedeutet ist. Diese Bewegungsverbindung kann beispielsweise durch eine Ultraschallschweißung oder dgl. hergestellt werden. Es ist aber auch möglich, den Stützteil 11 in den Basisteil 22 einzukleben oder die beiden Teile mit gegengleichen Rippungen zu versehen, sodaß diese gegen eine Relativverdrehung gehaltert sind. Die Positionierung in Längsrichtung der Spritzennadel 3 erfolgt mittels der Haltevorrichtung 6, die durch fingerartige Klauen gebildet wird, wie sie beispielsweise in der WO-A-86/03126 des gleichen Anmelders beschrieben ist. Gleichermaßen kann auch der Sollbruch bereich 24 sowie die Nadelschutzkappe 5 entsprechend den dort beschriebenen Ausführungsbeispielen ausgebildet sein.

Wie aus den Fig.1 bis 3 weiters ersichtlich ist, ragt die Spritzennadel 3 über eine Länge 25 über eine Stirnseite 26 des Lagerteiles 12 vor. Diese Länge 25 ist größer als eine Distanz 26' zwischen einem der Dichtscheite 10 zugeordneten stirnseitigen Ende des Stützteiles 11 und dessen Stirnseite 7. Dadurch wird sichergestellt, daß nach dem vollständigen Eindrehen des Lagerteiles 12 in den Stützteil 11 die Verschlußvorrichtung 9, z.B. die Dichtscheibe aus Pharmagummi, mit dem vom Ende 16 abgekehrten rückwärtigen Ende 27 der Spritzennadel 3 durchstoßen wird.

Die Position, in der der Lagerteil 12 zur Gänze in den Stützteil 11 eingedreht ist, ist am besten aus Fig.2 zu ersehen und hieraus ist auch zu ersehen, daß in diesem Fall das Ende 27 der Spritzennadel 3 bereits die Dichtscheibe 10, also den Pharmagummi, durchstoßen hat und somit die im Spritzenkörper 2 schematisch angedeutete lösung 28 direkt in einen Spritzennadelkanal 29 der Spritzennadel 3 eintreten kann.

Zum Öffnen des die Öffnungsvorrichtung bildenden Soll bruchbereiches 24 wird folgendermaßen vorgegangen:

Von dem Benutzer der Injektionsspritze 1 wird diese in eine Hand genommen, wobei der Spritzenkörper 2 und der Basisteil 22 der Nadelschutzkappe 5 erfaßt wird. Um ein rutschfreies Ergreifen des Basisteiles 22 zu ermöglichen, kann dieser - wie schematisch angedeutet - mit einer Längsrippung bzw. Riffelung versehen sein. Mit der anderen Hand werden an den Kappenteil 18 der Nadelschutzkappe 5 angeformte Laschen 30 erfaßt und der Kappenteil 18 gegenüber dem Basisteil 22 um eine Längsachse der Spritzennadel 3 verdreht, wie dies mit einem Pfeil 31 angedeutet ist. Dadurch wird eine Wand 32 im Sollbruchbereich 24 - wie in Fig.2 schematisch angedeutet - zerbrochen, worauf der Kappenteil 18 solange in Richtung des Pfeiles 31 weitergedreht wird, bis das Ende 27 der Spritzennadel 3 die Dichtscheibe 10 durchstoßen hat bzw. da der Kunststoff des Basisteiles 22 der Nadelschutzkappe 5 meist undurchsichtig ist, bis der Lagerteil 12 mit seiner Stirnseite 26 am Stützteil 11 ansteht bzw. das Innengewinde 15 zu Ende ist. Wie ersichtlich, kommt es dabei zu einer Längsbewegung des Lagerteiles 12 bzw. des Fortsatzes 17 entlang der Mitnahmeleisten 21 im Kappenteil 18 der Nadelschutzkappe 5, wie dies mit einem Pfeil 33 schematisch angedeutet ist. Samit wird durch eine Drehbewegung des Kappenteiles 18 der Nadelschutzkappe 5 im Sinne des Pfeiles 31 eine Längsbewegung der Spritzennadel 3 bewirkt und es kann damit die Verschlußvorrichtung 9 geöffnet werden.

Diese Verschlußvorrichtung 9 kann natürlich selbstverständlich anstelle der gezeigten, aus Pharmagummi bestehenden Dichtscheibe, durch eine beim Herstellen des Stützteiles 11 angefertigte Haut im Bereich einer Bohrung 34 des Stützteiles 11 erfolgen. Es ist beispielsweise aber auch möglich, eine kleinere Dichtscheibe in die Form einzulegen und in den Stützteil 11 direkt einzuspritzen. Vorteilhaft ist in jedem Fall, wenn die Verschlußvorrichtung 9 gleichzeitig auch die Dichtvorrichtung zwischen dem Spritzenkörper 2 und dem Stützteil 11 des Nadelträgers 4 bildet.

Wie aus Fig.3 ersichtlich ist, sind beim vorliegenden Ausführungs beispiel vier Mitnahmeleisten 21 und dementsprechend auch vier nutförmige Vertiefungen 19 vorgesehen. Anstelle dessen ist es selbstverständlich auch möglich, lediglich eine, zwei, drei oder auch mehrere solche Mitnahmeleisten 21 anzuordnen.

Gleichermaßen ist es auch möglich, den inneren Querschnitt des Kappenteiles 18 mehreckig auszubilden und dem Lagerteil 12 einen entsprechenden Außenquerschnitt zu geben, wodurch ebenfalls eine Mitnahme des Lagerteiles 12 beim Verdrehen des Kappenteiles 18 im Sinne des Pfeiles 31 erzielt werden kann.

Das Öffnungsorgan wird bei der vorliegenden Erfindung durch den Lagerteil 12 in Verbindung mit dem Ende 27 der Spritzennadel 3 gebildet. Die Vorteile dieser Ausbildung der Öffnungsvorrichtung können auch dann erreicht werden, wenn die Betätigung des Lagerteiles 12 nicht mittels des Kappenteiles 18 der Nadelschutzkappe, sondern bei spiel sweise nach deren Entfernung direkt durch manuellen Eingriff erfolgt.

Desweiteren ist auch die Anordnung und Ausbildung der Mitnahmevorrichtung 20, unabhängig von der speziellen Gestaltung der Öffnungsvorrichtung bzw. der zwischen dem Lagerteil und dem Stützteil angeordneten Führungsvorrichtung möglich.

## Patentansprüche

1. Injektionsspritze mit einem Spritzenkörper (2) und einem in diesen eingesetzten, die Spritzennadel (3) aufnehmenden Nadelträger (4) und einer mit dem Nadelträger (4) kraft- und bzw. oder formschlüssig verbundenen, mit einem Kupplungsteil des Spritzenkörpers (2) zumindest teilweise formschlüssig kuppelbaren Haltevorrichtung (6) und einer die Spritzennadel (3) umhüllenden und sich bis in den Bereich des Nadelträgers (4) erstreckenden Nadelschutzkappe (5) und einer zwischen dem Nadelträger (4) und dem der Nadelspitze (16) zugeordneten Ende der Nadelschutzkappe (5) angeordneten Öffnungsvorrichtung (24) sowie einer zwischen dem Spritzenkörper (2) und dem von diesem abgewendeten Ende der Spritzennadel (3) angeordneten Verschlußvorrichtung (3) für einen Spritzennadel, der ein durch die Spritzennadel gebildetes Öffnungsorgan (27) zugeordnet ist, das in einer dem Nadelträger (4) zugeordneten Führungsvorrichtung (13) verstellbar gelagert ist, dadurch gekennzeichnet, daß die Haltevorrichtung (6) mit einem Stützteil (11) des Nadelträgers (4) kraft- und bzw. oder formschlüssig bewegungsverbunden ist und die Führungsvorrichtung (13) zwischen dem Stützteil (11) und einem die Spritzennadel (3) aufnehmenden Lagerteil (12), insbesondere durch ein Gewinde, gebildet ist.

2. Injektionsspritze nach Anspruch 1, dadurch gekennzeichnet, daß der Stützteil (11) mit einem Basisteil (22) der Nadelschutzkappe (5) bewegungsverbunden ist.

3. Injektionsspritze nach einem der Ansprüche 1 oder 2, dadurch gekennzeichnet, daß sich der Stützteil (11) von dem Basisteil (22) der Nadelschutzkappe (5) über die Öffnungsvorrichtung (23) bis in den Bereich eines Kappenteiles (18) erstreckt und daß die drehfeste Verbindung zwischen dem Basisteil (22) und dem Stützteil (11) zwischen der Öffnungsvorrichtung (23) und der Verschlußvorrichtung (9) angeordnet ist und der in Richtung der Nadelspitze der Spritzennadel (3) anschließenden Bereich des Stützteiles (11) einen Führungsteil für den drehbaren Kappenteil (18) - Pfeil 31 - bildet.

**EP 0 314 696 B1**

4. Injektionsspritze nach einem der Ansprüche 1 bis 3, dadurch gekennzeichnet, daß eine Länge der (25) über den Lagerteil (12) in Richtung der Verschlußvorrichtung (9) vorspringenden Spritzennadel (3) größer ist als eine Distanz (26') zwischen dem dem Spritzenkörper (2) zugewandten Ende der Führungsvorrichtung (13) im Stützteil (11) und der Verschlußvorrichtung (9), insbesondere der Dichtscheibe (10).

5. Injektionsspritze nach einem der Ansprüche 1 bis 4, dadurch gekennzeichnet, daß eine Mitnahmevorrichtung (20) für den Lagerteil (12) der Spritzennadel (3) im Kappenteil (18) angeordnet, die vorzugsweise durch über eine dem Lagerteil (12) zugewandte Oberfläches des Kappenteiles (18) vorspringende und in Nuten des Lagerteils (12) eingreifende Mitnahmeleisten (21) gebildet ist.

6. Injektionsspritze nach einem der Ansprüche 1 bis 5, dadurch gekennzeichnet, daß die Mitnahmevorrichtung (20) durch den Kappenteil (18) der Nadelschutzkappe (5) gebildet ist und einen mehreckigen Innenquerschnitt aufweist, wobei der dem Kappenteil (18) zugeordnete Lagerteil (12) eine äußere Querschnittsform aufweist, die der inneren Querschnittsform des Kappenteiles (18) im wesentlichen entspricht.

## Claims

1. Injection syringe comprising a syringe body (2) and a needle carrier (4) inset therein and receiving the syringe needle (3) and a securing device (6) which is connected to the needle carrier (4) in a force-locked and/or shape-locked manner and is connectible in at least partially shape-locked manner to a coupling member of the syringe body (2), and a needle protection cap (5) sheathing the syringe needle (3) and extending into the area of the needle carrier (4), and an opening device (24) placed between the needle carrier (4) and the extremity of the needle protection cap (5) associated with the needle tip (16) as well as a closing or sealing device (9) for a syringe passage arranged between the syringe body (2) and the extremity of the syringe needle (3) facing away from the same, with which an opening element (27) is associated formed by the syringe needle (3) which is displaceable arranged with respect to a guiding device (13) associated with the needle carrier (4) characterised in that supporting section (11) of the needle carrier (4) is joined to the securing device (6) in force-locked and/or shape-locked manner and the guiding device (13) is situated between a supporting section (11) and a bearing section (12) receiving the syringe needle (3), and is formed by a screw-thread in particular.

2. Injection syringe according to claim 1, characterised in that the supporting section (11) is coupled in motion with a base section (22) of the needle protection cap (5) coupled thereto.

3. Injection syringe according to claims 1 or 2, characterised in that the supporting section (11) extends from the base section (22) of the needle protection cap (5) over the opening device (23) and into the area of a cap section (18) and that the co-rotatory connection between the base section (22) and the supporting section (11) being situated between the opening device (23) and the closing device (9) and the area of the supporting section (11) following in the direction of the needle tip of the syringe needle (3) forms a guiding element for the rotatable cap section (18) - arrow (31).

4. Injection device according to one of the claims 1 to 3, characterised in that a length (25) of the syringe needle (3) projecting beyond the bearing section (12) in the direction of the closing device (9) is greater than a spacing (26') between the extremity facing towards the syringe body (2) of the guding device (13) in the supporting section (11) and the closing device (9), being the sealing disc (10) in particular.

5. Injection syringe according to one of the claims 1 to 4, characterised in that the entraining device (20) for the bearing section (12) of the syringe needle (3) is arranged in the cap section (18) and is in particular formed by entraining fins (21) which projects beyond a surface of the cap section (18) facing towards the bearing section (12) and engaging into the grooves.

6. Injection syringe according to one of the claims 1 to 5, characterised in that the entraining device (20) is formed by the cap section (18) of the needle protection cap (5) and has a polygonal internal cross-section, the bearing section associated with the cap section (18) having an external cross-sectional shape which substantially corresponds to the internal cross-sectional shape of the cap section (18).

## Revendications

1. Seringue d'injection avec un corps de seringue (2) et un porte-aiguille (4) monté dans celui-ci et recevant l'aiguille d'injection (3) et un dispositif de maintien (6) relié au porte-aiguille (4) par liaison par force et/ou par forme et accouplable au moins partiellement par liaison par forme avec un élément d'accouplement du corps de seringue (2), et un capuchon de protection d'aiguille (5) enveloppant l'aiguille d'injection (3) et s'étendant jusqu'au voisinage du porte-aiguille (4), et un dispositif d'ouverture (24) disposé entre le porte-aiguille (4) et l'extrémité du capuchon de protection d'aiguille (5) associée à la pointe d'aiguille (16) ainsi qu'avec un dispositif de fermeture (9) pour un canal de seringue, disposé entre le corps de seringue (2) et l'extrémité, tournée à l'opposé de celui-ci, de l'aiguille d'injection (3), lequel canal est associé à un organe d'ouverture (27) formé par l'aiguille d'injection (3) et qui est monté de façon déplaçable dans un dispositif de guidage (13) associé au porte-aiguille (4), caractérisée en ce que le dispositif de maintien (6) est relié en mouvement par liaison par force et/ou par forme à un élément d'appui (11) du porte-aiguille (4) et le dispositif de guidage (13) est formé, entre l'élément d'appui (11) et un élément formant palier (12) recevant l'aiguille d'injection (3), en particulier par un filetage.

2. Seringue d'injection selon la revendication 1, caractérisée en ce que l'élément d'appui (11) est relié en mouvement à un élément de base (22) du capuchon de protection d'aiguille (5).

3. Seringue d'injection selon l'une des revendications 1 ou 2, caractérisée en ce que l'élément d'appui (11) s'étend depuis l'élément de base (22) du capuchon de protection d'aiguille (5) par-dessus le dispositif d'ouverture (23) jusqu'au voisinage d'un élément de capuchon (18) et en ce que la liaison solidaire en rotation entre l'élément de base (22) et l'élément d'appui (11) est disposée entre le dispositif d'ouverture (23) et le dispositif de fermeture (9) et la portion de l'élément d'appui (11), adjacente en direction de la pointe d'aiguille de l'aiguille d'injection (3), forme un élément de guidage pour l'élément de capuchon rotatif (18) - flèche 31 -.

4. Seringue d'injection selon l'une des revendications 1 à 3, caractérisée en ce qu'une longueur (25) de l'aiguille d'injection (3), s'étendant en saillie au-delà de l'élément formant palier (12) en direction du dispositif de fermeture (9), est plus grande qu'une distance (26') entre l'extrémité, tournée vers le corps de seringue (2), du dispositif de guidage (13) dans l'élément d'appui (11) et le dispositif de fermeture (9), en particulier le disque d'étanchéité (10).

5. Seringue d'injection selon une des revendications 1 à 4, caractérisée en ce qu'un dispositif entraîneur (20) pour l'élément formant palier (12) de l'aiguille d'injection (3) est disposé dans l'élément de capuchon (18), lequel dispositif est de préférence formé par des nervures entraîneuses (21) s'étendant en saillie au-delà d'une surface de l'élément de capuchon (18) tournée vers l'élément formant palier (12) et pénétrant dans des rainures de l'élément formant palier (12).

6. Seringue d'injection selon une des revendications 1 à 5, caractérisée en ce que le dispositif entraîneur (20) est formé par l'élément de capuchon (18) du capuchon de protection d'aiguille (5) et présente une section transversale interne polygonale, l'élément formant palier (12), associé à l'élément de capuchon (18), présentant une forme de section transversale extérieure qui correspond sensiblement à la forme de section transversale intérieure de l'élément de capuchon (18).

**FIG 1**

**FIG 2**

**FIG 3**